# EUROPEAN PATENT SPECIFICATION

(11) **EP 2 838 470 B1**
(45) Date of publication and mention of the grant of the patent: **13.06.2018**
(21) Application number: 13778653.9
(22) Date of filing: 18.04.2013
(51) Int. Cl.: A61F 9/007

(54) **DELIVERY SYSTEM FOR OCULAR IMPLANT**
ABGABESYSTEM FÜR EIN AUGENIMPLANTAT
SYSTÈME DE POSE POUR UN IMPLANT OCULAIRE

(30) Priority: 19.04.2012 US 201261635471 P; 24.04.2012 US 201261637789 P
(43) Date of publication of application: 25.02.2015
(60) Divisional application: 18164159.8
(73) Proprietor: NOVARTIS AG, 4056 Basel (CH)
(72) Inventor: SCHALLER, Michael, Menlo Park, CA 94025 (US); LARI, David, Menlo Park, CA 94025 (US); CLAUSON, Luke, Menlo Park, CA 94025 (US); WHITE, Nathan, Menlo Park, CA 94025 (US); LILLY, Richard, S., Menlo Park, CA 94025 (US); NEWELL, Matthew, Menlo Park, CA 94025 (US)
(74) Representative: Kazi, Ilya
(86) International application number: PCT/US2013/037234
(87) International publication number: WO 2013/158919

(56) References cited:
- WO-A1-96/36377
- WO-A1-2010/065970
- WO-A1-2010/115101
- WO-A2-2004/026106
- WO-A2-2004/026106
- US-A1- 2009 182 421
- US-A1- 2011 098 629
- US-A1- 2011 098 809
- US-A1- 2011 112 546

## Description

### BACKGROUND

This disclosure relates generally to methods and devices for use in delivering devices for treating glaucoma. An exemplary implant system and method is disclosed in U.S. Patent Publication No. 2011-0098809.

The mechanisms that cause glaucoma are not completely known. It is known that glaucoma results in abnormally high pressure in the eye, which leads to optic nerve damage. Overtime, the increased pressure can cause damage to the optic nerve, which can lead to blindness. Treatment strategies have focused on keeping the intraocular pressure down in order to preserve as much vision as possible over the remainder of the patient's life.

Pursuant to such strategies, one or more implants can be delivered into the eye for shunting fluid out of the anterior chamber in order to regulate pressure in the eye. Accurate placement of an implant in the angle of the eye is critical for the targeted effect of reducing intraocular pressure (IOP). Placing an implant too distally into the eye, such as too distally into the supraciliary space, may leave no portion of the implant remaining in the anterior chamber. This may inhibit aqueous outflow, as the fluid will not have a direct communication with the flow target location if there is no opening to the anterior chamber.

Conversely if the implant is placed too proximally in the supraciliary space such that a significant portion of the implant remains in the anterior chamber, damage to the corneal endothelium may result from implants that protrude upwards and touch the cornea. Implants placed too proximally may also touch the iris resulting in increased amounts of pigment dispersion in the eye, which can increase outflow resistance and intraocular pressure by clogging the trabecular meshwork. Correct placement of the implant is desired for a safe and successful surgical outcome.

In view of the foregoing, there is a need for improved delivery systems for delivering implants into the eye such as by way of an *ab inferno* procedure.

### SUMMARY

There is a need for improved delivery systems, devices and methods for the treatment of eye diseases such as glaucoma. The invention is defined by claim 1.

In a first embodiment, disclosed herein is a delivery device for delivering an ocular implant into an eye. The delivery device can include a proximal handle portion and a distal delivery portion coupled to a distal end of the handle portion and configured to releasably hold an ocular implant. In addition, the delivery portion can include a sheath positioned axially over a guidewire. The delivery device can further include an actuator coupled to a mechanism that releases the ocular implant from the delivery portion upon actuation of the actuator.

Also described herein are methods of delivering an ocular implant to a target location within an eye. In an embodiment, disclosed is a method including loading the ocular implant onto a distal delivery portion of a delivery system. The delivery system can include a proximal handle portion with the delivery portion coupled to a distal end of the handle portion. In addition, the delivery portion can be configured to releasably hold the ocular implant. The delivery portion can further include a sheath positioned axially over a guidewire. Additionally, the delivery device can include an actuator coupled to a mechanism that releases the ocular implant from the delivery portion upon actuation of the actuator. The method can further include inserting the distal delivery portion and the ocular implant into the eye through a corneal incision and positioning the ocular implant into the target location within the eye by way of an ab-interno procedure. Furthermore, the method can include actuating the actuator and releasing the ocular implant into the target location.

Other features and advantages should be apparent from the following description of various embodiments, which illustrate, by way of example, the principles of the described subject matter.

### BRIEF DESCRIPTION OF THE DRAWINGS

These and other aspects will now be described in detail with reference to the following drawings.
FIG. 1 shows an example cross-sectional view of a portion of the human eye.
FIG. 2 shows and an example partial cross-sectional view of the eye showing a part of the anterior and posterior chambers of the eye and an ocular implant implanted in the eye.
FIG. 3 shows a perspective view of an embodiment of a delivery device having a proximal handle component and a distal delivery component with an ocular implant loaded onto the distal delivery component.
FIG. 4 shows a close up view of the distal end of the delivery component of FIG. 3 which illustrates the implant loaded onto a guidewire of the delivery system.
FIG. 5 shows a partial cross section view of the delivery system of FIG. 3 showing a distal portion of the handle component, including the spring-loaded actuator in a compressed configuration, and the distal delivery component.
FIG. 6 shows the partial cross section view of the delivery system of FIG. 5 with the spring-loaded actuator shown in a decompressed configuration which releases the implant from the distal delivery component.
FIG. 7 shows an embodiment of the guidewire of the delivery system having a curved configuration.
FIG. 8 shows an embodiment of the guidewire of the delivery system having a sinusoidal configuration.
FIG. 9 shows an embodiment of the guidewire of the delivery system having a length sufficient to extend from the supraciliary space down to the sub-retinal space.
FIG. 10 shows an enlarged view of the anterior region of the eye with the implant approaching the supraciliary space or suprachoroidal space from the anterior chamber.
FIG. 11 shows a perspective view of an embodiment of a direct visualization (DV) system.
FIG. 12 shows an enlarged view of a distal end of the DV system including a part of a DV wire 12 and stopper tube 16.
FIG. 13 shows a cross-sectional view of a portion of the DV system shown in FIG. 11.
FIG. 14 shows the distal end of the DV system shown in FIG. 11 inserted into an eye.
FIG. 15 shows the DV wire of the DV system aligned alongside an implant delivery applier showing the corresponding indicators.
FIG. 16 shows the DV wire inserted into the eye for measuring anatomical features of the eye.
FIG. 17 shows the distal end of the DV wire abutting the base of the angle of the eye and the stopper tube in an advanced position along the DV wire.
FIG. 18 shows the implant delivery applier implanting an ocular implant through the same incision the DV system used in FIGS. 8 and 9.
FIG. 19 shows the indicators on the implant delivery applier being used to determine the proper insertion depth of the implant.
FIG. 20 shows the implant in an implanted state and providing fluid communication between the anterior chamber and the suprachoroidal or supraciliary space.
FIGS. 21A shows an embodiment of the implant delivery applier having a feedback mechanism.
FIGS. 21B shows the feedback mechanism of the implant delivery applier shown in FIG. 21A in a retracted state.
FIG. 22 shows a cross-section view of an embodiment of a pencap implant loader configured to house an implant and releaseably couple to a delivery device.
FIG. 23 shows a perspective view of the pencap implant loader of FIG. 22.
FIG. 24 shows a cross-section view of another embodiment of a pencap implant loader configured to house an implant and releaseably couple to a delivery device.
FIG. 25 shows a perspective view of the pencap implant loader of FIG. 24.

Like reference symbols in the various drawings indicate like elements.

### DETAILED DESCRIPTION

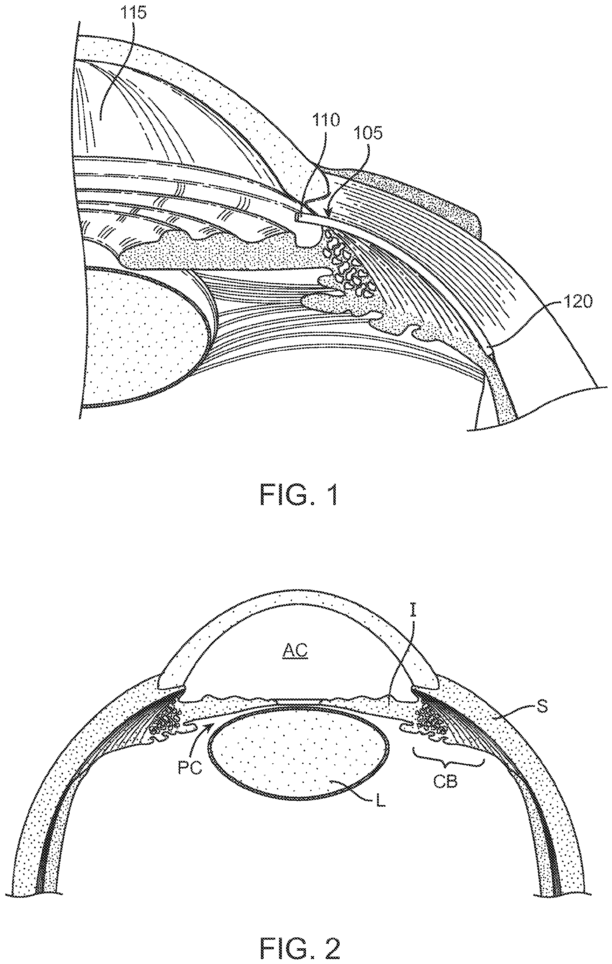

FIG. 1 is a cross-sectional, perspective view of a portion of the eye showing the anterior and posterior chambers of the eye. A schematic representation of an implant 105 is positioned inside the eye such that a proximal end 110 is located in the anterior chamber 115 and a distal end 120 communicates with and/or is located in or near the supraciliary space or suprachoroidal space (sometimes referred to as the perichoroidal space). It should be appreciated that FIG. 1 and other figures herein are schematic and are not necessarily to scale with respect to size and relative positions of actual eye tissue.

The implant 105 provides a fluid pathway between the anterior chamber 115 into the supraciliary space and toward the suprachoroidal space. The implant 105 has a distal end120 that may be positioned in the supraciliary space or the suprachoroidal space. The implant 105 may be positioned at least partially between the ciliary body and the sclera or it may be at least partially positioned between the sclera and the choroid. The distal end 120 of the implant 105 is not necessarily positioned between the choroid and the sclera.

In an embodiment, the implant 105 is an elongate element having one or more internal lumens through which aqueous humor can flow from the anterior chamber 115 into the supraciliary space. The implant 105 can have a substantially uniform internal diameter along its entire length, although the shape of the implant 105 can vary along its length (either before or after insertion of the implant), as described below. Moreover, the implant 105 can have various cross-sectional shapes (such as a circular, oval or rectangular shape) and can vary in cross-sectional shape moving along its length. The cross-sectional shape can be selected to facilitate easy insertion into the eye. The following applications describe exemplary implants: U.S. Patent Publication Nos. 2007-0191863 and 2009-0182421.

FIG. 2 is a cross-sectional view of a portion of the human eye. The eye is generally spherical and is covered on the outside by the sclera S. The retina (not shown) lines the inside posterior half of the eye. The retina registers the light and sends signals to the brain via the optic nerve. The bulk of the eye is filled and supported by the vitreous body, a clear, jelly-like substance. The elastic lens L is located near the front of the eye. The lens L provides adjustment of focus and is suspended within a capsular bag from the ciliary body CB, which contains the muscles that change the focal length of the lens. A volume in front of the lens L is divided into two by the iris I, which controls the aperture of the lens and the amount of light striking the retina. The pupil is a hole in the center of the iris I through which light passes. The volume between the iris I and the lens L is the posterior chamber PC. The volume between the iris I and the cornea is the anterior chamber AC. Both chambers are filled with a clear liquid known as aqueous humor.

The ciliary body CB continuously forms aqueous humor in the posterior chamber PC by secretion from the blood vessels. The aqueous humor flows around the lens L and iris I into the anterior chamber and exits the eye through the trabecular meshwork, a sieve-like structure situated at the corner of the iris I and the wall of the eye (the corner is known as the iridocorneal angle). Some of the aqueous humor filters through the trabecular meshwork near the iris root into Schlemm's canal, a small channel that drains into the ocular veins. A smaller portion rejoins the venous circulation after passing through the ciliary body and eventually through the sclera (the uveoscleral route).

The internal lumen of the implant 105 serves as a passageway for the flow of aqueous humor through the implant 105 directly from the anterior chamber toward or into the supraciliary or suprachoroidal space. In addition, the internal lumen of the implant 105 can be used as an access location to mount the implant 105 onto a delivery device, as described in more detail below. The internal lumen can also be used as a pathway for flowing fluid, such as an irrigation fluid or a visco-elastic substance(s), into the eye for flushing or to maintain pressure in the anterior chamber, or using the fluid to assist in dissection, visualization or hydraulic creation of a dissection plane into or within the suprachoroidal space.

Fluid can be flowed toward or into the supraciliary or suprachoroidal space, for example via a delivery cannula or through the internal lumen of the shunt. The fluid can be flowed into the eye with a pressure sufficient to form a dissection plane into or within the supraciliary suprachoroidal space. The fluid can accumulate within the eye so as to form a lake. In general, hydro-dissection or the injection of fluids such as a visco-elastic substance(s) can be used to separate the ciliary body from the sclera to enlarge an area of detachment of the ciliary body from the sclera with or without insertion of a device.

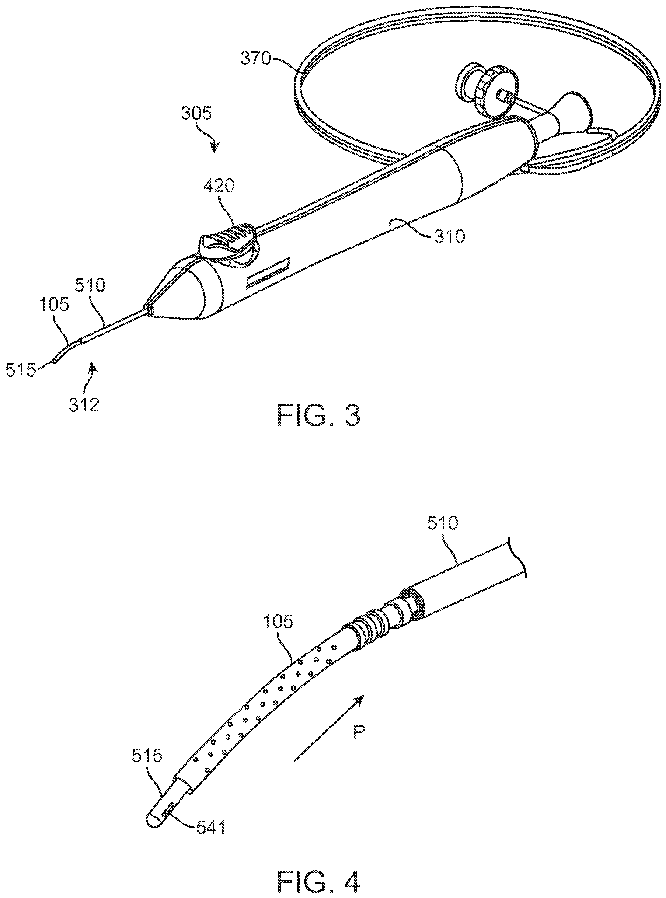

FIG. 3 shows an embodiment of a delivery system 305 that can be used to deliver the implant 105 into the eye. In some embodiments, the implant 105 can provide fluid communication between the anterior chamber toward the suprachoroidal or supraciliary space while in an implanted state. It should be appreciated that these delivery systems 305 are exemplary and that variations in the structure, shape and actuation of the delivery system 305 are possible. The delivery system 305 can include a proximal handle component 310 and a distal delivery component 312. The proximal handle component 310 can include an actuator 420, such as a button, to control the release of an implant from the delivery component 312 into a target location in the eye. The actuator 420 can vary in structure and is not limited to a button.

An embodiment of the delivery component 312 includes an elongate applier in the form of a guidewire 515 and a "stopper" or sheath 510 positioned axially over the guidewire 515. The guidewire 515 can insert longitudinally through the internal lumen of the implant 105 and can assist in inserting and positioning the implant 105 into the target location. The sheath 510 can aid in the release of the implant 105 from the delivery component 312 into the target location in the eye. In addition, the actuator 420 can be used to control movement or relative movement of the guidewire 515 and/or the sheath 510. For example, the sheath 510 can be fixed relative to the handle component 310 and act as a stopper which can impede the implant 105 from moving in a proximal direction as the guidewire 515 is withdrawn proximally from the implant 105 upon actuation of the actuator 420.

For example, in a first state, the guidewire 515 can be extended distally relative to a distal end of the sheath 510. Actuation of the actuator 420, such as by pressing the actuator 420, can cause the guidewire 515 to slide proximally or retract into the sheath 510. This can effectively disengage the implant 105 off the distal end of the guidewire 515 and releases the implant 105 in a controlled fashion into the target location. Controlled disengagement of the implant 105 off the distal end of the guidewire 515 can assist in ensuring that positioning of the implant 105 within the target location is maintained.

FIG. 4 shows an embodiment of the implant 105 mounted on the delivery component 312 of the delivery system 305. More specifically, the implant 105 can be mounted on the distal region of the guidewire 515, as shown in FIG. 4. In addition, the sheath 510 can be sized and shaped to receive or abut a portion of the proximal end of the implant 105. In this embodiment, upon actuation of the actuator 420, the guidewire 515 can slide in a proximal direction (arrow P) into the sheath 510 which can allow the proximal end of the implant 105 to abut the distal end of the sheath 510 and prevent the implant 105 from sliding in the proximal direction. This can effectively disengage the implant 105 off the distal end of the guidewire 515 and controllably releases the implant 105 into the target location within the eye.

In some embodiments, the actuator 420 can be a push-button that is coupled to a spring-activated mechanism. Upon applying a force onto the actuator 420, the spring mechanism can retract the guidewire 515 toward and/or into the sheath 510 which can release the implant 105 from the guidewire 515. The mechanism by which the guidewire 515 can be withdrawn into the sheath 510 can be a spring activated assembly or any of a variety of mechanisms that allow the guidewire to retract upon activation of an actuator.

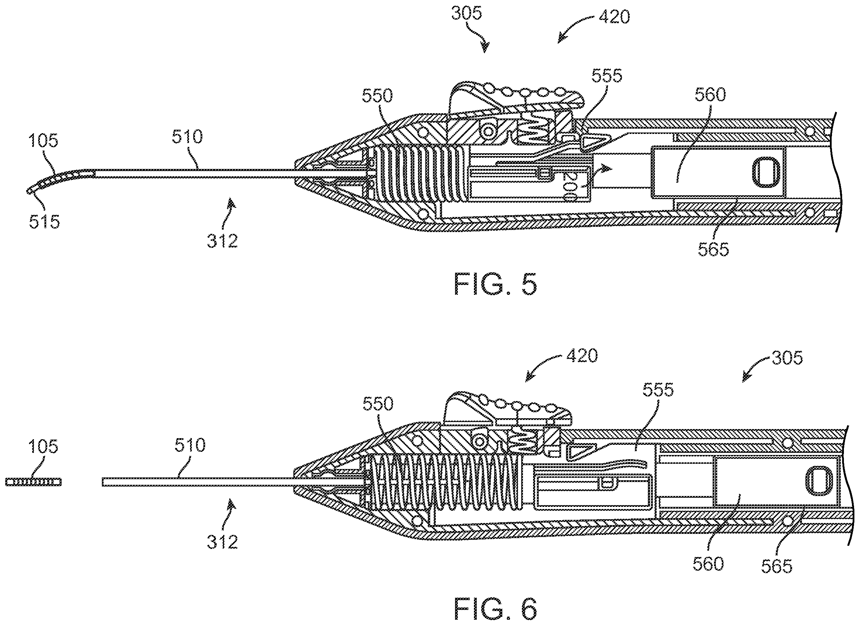

FIG. 5 shows an embodiment of a portion of the delivery system 305 in cross-section with the implant 105 loaded onto the guidewire 515. The delivery system 305 includes a front spring 550 which can assist in positioning the guidewire 515. For example, the front spring 550 can be compressed or charged which can allow the guidewire 515 to be positioned in an extended state relative to the handle 310. When the guidewire 515 is in an extended state, the guidewire 515 can be loaded with the implant 105, as shown in FIG. 5.

The delivery system 305 can include a variety of mechanisms for assisting in the positioning of the guidewire 515. For example, the delivery system 305 can include a feature which can interact with the actuator 420 in order to allow the actuator to assist in positioning the guidewire 515. For example, the guidewire 515 can be attached at a proximal end to a piston 560 having a de-tent latch 555. The de-tent latch 555 can interact with the actuator 420 such that upon actuation of the actuator 420, the 555 latch can release the piston 560 from a locked position and allow the piston 560 to move. For example, once the piston 560 is allowed to move, the front spring 550 can force the piston to move in a direction, such as in a proximal direction, thus causing the guidewire 515 to move in a proximal direction. Movement of the guidewire 515 in a proximal direction can allow the implant 105 loaded on the distal end of the guidewire 515 to be released from the guidewire 515.

In some embodiments, the actuator 420 can be configured such that when actuated or depressed by the user, the detent latch 555 of the piston 560 is flexed downward thereby allowing the front spring 550 to release. As the piston 560 moves proximally with the guidewire 515, the implant 105 can abut the distal end of the stopper tube 510 and release from the guidewire 515. FIG. 6 shows an embodiment of the delivery system 305 in a retracted state where the front spring 550 is in a decompressed state with the implant 105 fully released from the guidewire 515.

The travel of the piston 560 can be defined such that the guidewire 515 reaches a complete stop in the proximal direction only after the implant 105 is fully released. In addition, the force of the front spring 550 can allow withdrawal of the guidewire 515 from the implant 105 when the implant 105 is positioned in a variety of angles relative to the stopper tube 510. For example, the force of the front spring 550 can allow the withdrawal of the guidewire 515 from the implant 105 when the implant 105 is at a 45 degree angle relative to the stopper tube 510, such as what may be encountered when the implant 105 is being deployed to the supraciliary space.

In some embodiments, for example, the front spring 550 can provide approximately 4.4 to 8.9 N (1 to 2 Ibf) at the compressed or charged configuration which can allow the guidewire 515 to withdraw from the implant 105, including when the implant 105 is positioned at an approximate 45 degree angle relative to the stopper tube 510. However, the front spring 550 can provide any of a variety of spring force which allows the guidewire 515 to release the implant 105 positioned at a variety of angles relative to at least the stopper tube 510.

In some embodiments, the front spring 550 can create approximately 8.9 to 44.5 N (2 to 10 Ibf). For example, a greater spring force of the front spring 550 can allow the guidewire 515 to retract in a variety of conditions. In addition, a lower force of the front spring, such as 0.4 to 4.4 N (0.1 to 1 Ibf) may reduce the speed of the retraction and reduce the force required to reload the system. Any of a variety of front springs 550 can be implemented in the delivery system 350.

A dampening element, such as grease 565, may be placed between the piston 560 and inside wall of the handle 310 which can assist in providing a slower retraction of the guidewire 515. A slower retraction of the guidewire 515 prevents or lessens any jerking motion of the delivery system 350 in the user's hands, including at the end of the piston 560 travel. This dampening grease 565 can be a silicone grease such that grease is unaffected by production level e-beam sterilization dose of 25 -50 kGy. In addition, other dampening elements aside from grease 565 may be used. Alternate dampening grease such as low, medium, or high viscosity fluorocarbons may be used to alter the dampening and speed of deployment. These materials may have a larger acceptable e-beam sterilization range.

In some embodiments, the spring-activated retraction of the guidewire 515 can improve the delivery of supraciliary and suprachoroidal implants. For example, some current tools for implanting ocular implants require a sliding motion of the user's finger, such as in the range of approximately 0.7112 cm (0.280" inches) of travel, in order to release the implant. The sliding motion can be difficult for surgeons to achieve while simultaneously holding the distal end of the delivery tool steady. In contrast, the spring activated mechanism of the present disclosure, including the spring activated push-button mechanism, allows for smaller and more ergonomic motion of the users finger to activate guidewire 515 retraction which also allows the user to maintain the distal end of the delivery device 312 in a steady position. In addition, the spring-activated mechanism of the present disclosure can allow implantation to occur more quickly and with less unwanted distal movement of the implant 105 during the guidewire retention.

The outer diameter of the guidewire 515 can be smaller than the inner diameter of the implant 105 (i.e. the fluid channel) such that the implant 105 can be loaded onto the guidewire 515 by sliding the guidewire 515 into and through an internal lumen of the implant 105. The guidewire 515 includes a retention feature that can act to retain the implant 105 on the guidewire 515. For example, the guidewire 515 can include a retention feature which can assist in retaining the implant 105 on the guidewire 515 during blunt dissection and implantation in order to prevent the implant 105 from inadvertently sliding off the guidewire 515.

Before the implant 105 has been released from the guidewire 515 and implanted into the target location within the eye, the implant 105 can be moved either distally or proximally in order to adjust its placement. This can exert axial forces on the implant 105 which may cause it to slip off the guidewire 515 if it is not well retained on the guidewire 515. Therefore, the guidewire 515 includes features which can assist in retaining the implant 105 onto the guidewire 515 during positioning of the implant 105, including positioning the implant 105 within the target location.

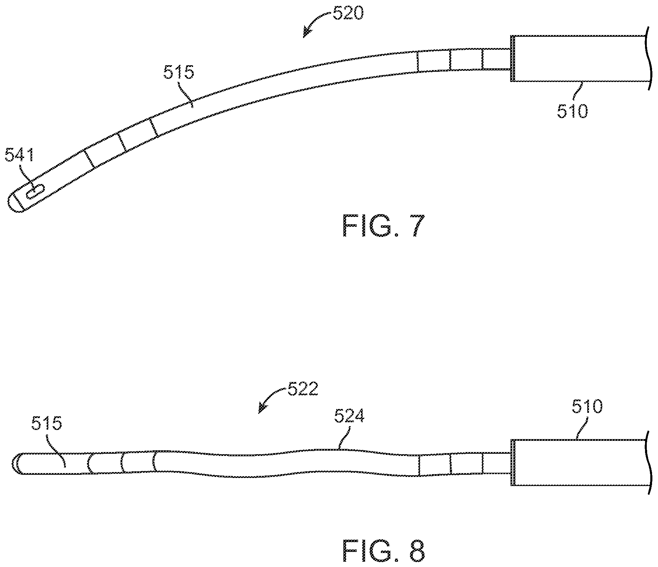

FIG. 7 shows an embodiment of a guidewire 515 which has at least one retention feature including a curved configuration 520 along a length of the guidewire 515. In some embodiments, the curved configuration 520 of the guidewire 515 can assist in facilitating entry of the implant 105 into the supracilliary space. In addition, the curvature of the guidewire 515 can change the shape of the implant 105 due to the implant 105 conforming to the curved shape of the guidewire 515 which can facilitate placement of the implant 105 into the supraciliary space as it curves along the scleral wall. The curvature radius or arc, including the curved configuration 520 of the guidewire 515, can vary and can be in the range of approximately 1.0795cm (.425") to about 1.3335 cm (.525") with a central angle of approximately 20 degrees to approximately 40 degrees.

Additionally, any part of the guidewire 515 can have the curved configuration 520, including either the distal end or the entire length of the guidewire 515. Furthermore, the guidewire 515 can alternate between having a variety of configurations, including both straight and curved configurations. For example, the guidewire 515 can have a curved configuration in its natural state but can conform to a straight passageway, such as through the handle 310 of the delivery system 305. Therefore, the guidewire 515 can conform to a straight passageway and return to a curved configuration after having passed through the straight passageway.

In some embodiments, the guidewire 515 can have one or more cut patters along a length of the guidewire 515 which can allow the guidewire 515 to be more flexible than the material comprising the guidewire 515 can allow. For example, the distal end or tip of the guidewire 515 can include a spiral cut pattern which allows the tip of the guidewire 515 to deflect or bend in one or more of a variety of directions relative to a longitudinal axis of the guidewire 515. Furthermore, the spiral cut pattern can allow the distal end or tip of the guidewire 515 to deflect or bend to a greater degree than what the guidewire could achieve without the spiral cut pattern. These cut patterns may additionally serve as fluid conduits which can provide a passageway for substances injected into the guidewire 515 to be released to an area surrounding the guidewire, including either the implant or the eye.

FIG. 8 shows an embodiment of the guidewire 515 according to the invention having at least one retention feature including a sinusoidal or S-curve configuration along a length of the guidewire 515. The sinusoidal or S-curve configuration can assist in retaining the implant 105 onto the guidewire 515, such as by at least one curved region 524 along a length of the guidewire 515. The at least one curved feature can include a protrusion, bump, etc. For example, the curved feature 524 can be configured to provide an interference fit between the guidewire 515 and the inner lumen of the implant 105.

In some embodiments, the retention feature can include an S-shaped curve along a length of the guidewire 515 which can have one or more rounded curved features 524, including bends or peaks, as shown in FIG. 8. Furthermore, each retention feature, such as curved feature 524, can form a point of contact between the inner lumen of the implant 105 and the guidewire 515. The curved features 524 of the guidewire S-curve can also reduce the risk of damaging the inner lumen of the implant 105 as the guidewire 515 is released from the implant 105. In addition, the retention features can provide a gentle interaction and retention between the guidewire 515 and the implant 105, including during removal of the guidewire 515 from the implant 105. Alternatively, the guidewire 515 retention features can be stamped, bent or shape-set, including in the shape of swells or other formations along at least a part of the length of the guidewire 515.

In an embodiment, an amount of retention force can be defined by the peak-to-peak distance between two or more retention features or curved features 524 of the implant 105. For example, larger peak-to-peak distances between the two or more curved features 524 can produce higher retention forces and smaller peak-to-peak distances can produce lower retention forces. In some embodiments, a peak-to-peak distance that is too large can cause damage to the implant 105, such as due to the guidewire 515 scraping away material along the inner lumen during removal. For example, the peak-to-peak distance may be in the range of approximately 0.0254 cm (0.0100") to approximately 0.0508 cm (0.0200"), or in the range of approximately 0.0305 cm (0.0120") to approximately 0.0381 cm (0.0150"). In addition, at least one retention force acting upon the implant 105, such as a polyimide implant, by the guidewire 515 of approximately .050 - .200 Ibf can be sufficient to retain the implant 105 along the guidewire 515 during manipulation of the implant 105 prior to implantation into the target location.

In alternate embodiments, the material of the guidewire 515 can be made out of one or more flexible materials, such as metals including stainless steel or elgiloy, and polymers such as Pebax, silicones, urethanes, including a variety of combinations of materials. In some embodiments, the guidewire 515 can have a radius of curvature or arc which is less than 1.0795cm (0.425"), such as in order to provide a small curvature of the implant 105 during insertion. This configuration can be advantageous when access between the incision and the target location requires the implant 105 to be introduced into the target location by way of a small radius, such as less than 1.0795cm (0.425").

Alternatively, the radius of curvature or arc of the guidewire 515 can be larger than 1.3335 cm (0.525"). Any of a variety of radius of curvature or arcs of the guidewire 515 can be implemented into any of the delivery systems 305 in order to best accommodate insertion of the implant 105 into the designated target location. For example, the radius of curvature or arc of the guidewire 515 may be such that it can allow the implant 105 to bend against the scleral wall during insertion into the supraciliary space. In addition, the retention features of the guidewire 515 can vary and can include one or more of a variety of shapes and sizes along a length of the guidewire 515. For example, the retention features can be configured to include spiral shapes, triangle peaks or the like. Additionally, the retention features can extend along one or more of a variety of planes, including more than one retention feature extending in planes positioned perpendicular relative to each other.

In addition, any number of retention features can be positioned along a length of the guidewire 515. For example, at least two, including more than five or more than ten retention features can be positioned along a length of the guidewire 515. In addition, each retention feature can provide the same or a variety of different amounts of retention forces for securing the implant 105 in a position along the guidewire 515. In some embodiments, the peak-to-peak distance between the retention features can be larger than the inner diameter of the implant 105 and can be a dimensioned larger than 0.0381 cm (.0150") such that it does not damage the implant 105.

In some embodiments of the delivery system 305, instead of using the guidewire 515 to provide retention of the implant 105, an additional feature of the delivery system 305 or device can be used in order to provide the necessary retention of the implant 105 onto the guidewire 515. This may include, for example, a Pebax material which can be coupled onto a part of the guidewire 515 in order to create at least a width along the guidewire 515 that is larger than the inner diameter of the implant 105. For example, the Pebax material can be crimped to the guidewire and can retain the implant 105 relative to the guidewire 515 until the implant 150 is released from the delivery system 305, such as after actuation of the actuator 420.

As shown in FIGS. 3 and 4, the delivery system 305 can include at least one fluid delivery feature which can be configured to deliver fluid into at least one of the implant or the eye, including during or after implantation of the implant 105. The delivered fluid can vary and may include a viscoelastic, drugs, stem cells, or a combination thereof. In addition, the delivery may be in combination with retinal or macula therapy.

The at least one fluid delivery feature can include an elongated tube 370 having at least one inner lumen. The elongated tube 370 can extend outward from the handle 310. In addition, the elongated tube 370 can extend through the handle 310. Additionally, the elongated tube 370 can have an internal lumen which communicates with an internal lumen of the guidewire 515.

In some embodiments, the guidewire 515 can include one or more outlet openings, such as slots 541 (FIG. 4), which can be located along a length of the guidewire 515, including along a distal region of the guidewire 515. The slots 541 can allow fluid communication between the internal lumen of the guidewire 515 and an area surrounding the guidewire 515. In addition, the outlet openings or slots 541 can also be in fluid communication with at least one inner lumen of the elongated tube 370.

In some embodiments, the elongated tube 370 can be connected at a proximal end to a source of fluid (such as via a Luer connection). The source of fluid can provide fluid into at least one inner lumen of the elongated tube 370 which can be delivered to a variety of places either within at least one of the delivery system 305, the implant 105 or the eye. For example, some of the fluid provided by the fluid source can be passed through the elongated tube 370 and exit the guidewire 515 via the slots 541 for delivery into the eye.

The size of the at least one inner lumens of the elongated tube 370 and guidewire 515 may vary. In an embodiment, the inner lumen of either the elongated tube 370 or guidewire 515 can be within a range of approximately 0.0025 cm (.001") to approximately 0.0254 cm (.010") in diameter, or approximately 0.0127cm (.005") to approximately 0.0229 cm (.009") in diameter. In addition, the size of the inner lumen can depend on the size constraints of the outer diameter of either the elongated tube 370 or the guidewire 515.

In some embodiments, the distal slots 541 of the guidewire 515 can allow fluid from at least the fluid source to be delivered to a distal end of the implant 105, including during or after implantation of the implant 105. In addition, fluid from the fluid source can be delivered to an area adjacent the distal end of the implant in order to create an aqueous lake or create a tenting effect around at least a part of or adjacent the implant 105. The size and location of the slots 541 can be sized, shaped and positioned along the guidewire 515 in order to create a variety of fluid delivery effects. For example, at least two slots 541 can be configured symmetrically relative to the distal end of the guidewire 515 which can allow the fluid to be delivered symmetrically around or near the distal end of the implant.

In an embodiment, the flow rate of the fluid from the fluid source can be within a range of approximately 1mg/sec to 10mg/sec, or approximately 2mg/sec to 5 mg/sec. In addition, the burst pressure of the delivery system 305, including the fluid delivery features, can be large enough to withstand the pressure of injecting a fluid through the lumens of the delivery system 305 and implants 105.

In some embodiments, the burst pressure of the delivery system 305 can be larger than the pressure required for the fluid to flow from the fluid source through at least the delivery system 305. For example, the burst pressure can be approximately 400 psi to approximately 1500 psi, or approximately 600 psi to approximately 1200 psi. In addition, the burst pressure required for viscoelastic flow of Healon 5 can be approximately 100 psi to approximately 500 psi, or approximately 200 psi to approximately 300 psi.

In some embodiments, fluid from the fluid source can be delivered to one or more sections along the axial length of the implant 105. For example, one or more holes along the length of the implant 105 (as shown in FIG. 4) can be configured to be sufficiently large such that a fluid may be delivered from the guidewire 515. For example, one or more slits 514 positioned along the length of the guidewire 515, such as below a loaded implant 105, can allow fluid to travel through the at least one hole along the length of the implant 105 and into the eye. For example, the fluid can flow out from the one or more holes along the length of the implant and into the supraciliary or suprachoroidal space surrounding the body of the implant 105 (depending on where the implant is positioned and the length of the implant). The release of fluid through the at least one hole along the length of the implant 105 can assist in creating additional space surrounding the implant 105 which can improve tenting.

One or more drugs can be delivered to the inner lumen of the implant 105 through the one or more holes or slits 514 along the axial length of the guidewire 515. Alternatively or in addition, drugs can be delivered through the guidewire 515 slots 541 positioned at or near the distal end of the guidewire 515 which can dispense fluid either before or during retraction of the guidewire 515. In some instances, this can reduce the fibrotic response of the surrounding tissue to the implant 105. Additionally, the delivery of fluids may be administered through separate components that do not retain the implant 105. For example, separate tubes may be inserted into the eye alongside of the implant 105 which can deliver drugs or viscoelastic to, for example, the distal end of the implant 105.

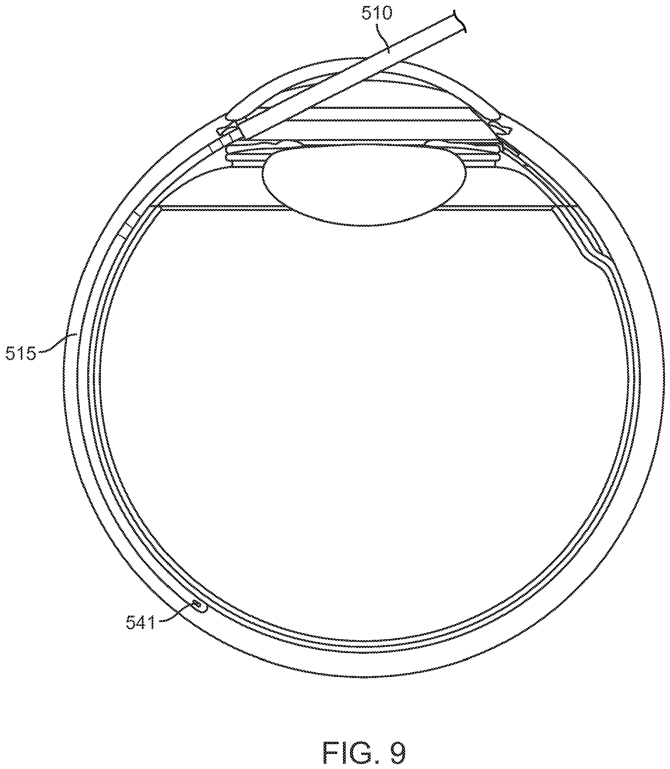

The system may also be used for the ab-interno delivery of fluids to other locations in the eye. FIG. 9, for example, shows the guidewire 515 having a length sufficient to extend from the supraciliary space down to the sub-retinal space. Fluid delivery in the subretinal portion of the eye may be advantageous because it can allow for direct delivery of drugs to the macula for diseases such as age related macular degeneration (AMD) or diabetic retinopathy, or the like. A variety of drugs can be delivered to the sub-retinal space, including anti-VEGF treatments or the like. Alternatively other fluids containing a stem cell therapeutic may be delivered through the guidewire 515 and into the sub-retinal or sub-macula space. These could be used to treat disease such as glaucoma, AMD, and diabetic retinopathy.

Additionally, fluid may be delivered to various anatomical structures comprising the eye. For example, fluid can be delivered to anatomical structures such as the Schlemm's Canal. By way of further example, the guidewire 515 can be passed through the Trabecular Meshwork, such as via an ab interno procedure, and into the Schlemm's Canal where viscoelastic substances can then be injected. The viscoelastic substances can then travel circumferentially around the eye for a number of hours which can dilate the Schlemm's Canal. In another embodiment, the guidewire 515 may be inserted through the sclera with the tip of the guidewire 515 just below the conjunctiva. Fluids such as viscoelastic may then be injected to create a sub-conjunctiva space which can form a filtration bleb.

A guidewire 515 assembly having an increased stiffness, such as one made from Nitinol, can be appropriately sized and delivered through an ab-interno approach. Alternate materials such as flexible polymers including Pebax, silicone, and urethane, can also be used. The ab-interno procedure can offer a patient significant reductions in complications and risks that are associated with the current ab-externo procedures, including conjunctivitis.

An example method of delivering and implanting the ocular implant 105 in the eye can include loading one or more implants 105 on a delivery system 305 and implanting the implants 105 by way of an ab interno procedure. The implant 105 can be implanted such that it can provide fluid communication between the anterior chamber and the supraciliary or suprachoroidal space. The implant 105 can then be secured in the eye so that it provides permanent fluid communication between the anterior chamber and the supraciliary space or suprachoroidal space.

The guidewire 515 can be positioned on the delivery system 305 such that the distal tip of the guidewire 515, the implant 105 and sheath 510 can penetrate through a small corneal incision in order to access the anterior chamber, such as along the limbus of the cornea. In an embodiment, the incision can be very close to the limbus, such as either at the level of the limbus or within 2 mm of the limbus in the clear cornea. The guidewire 515 can be used to make the incision or a separate cutting device can be used. For example, a knife-tipped device or diamond knife can be used to initially enter the cornea.

The corneal incision can have a size that is sufficient to permit passage of at least the implant 105. In an embodiment, the incision can be approximately 1 mm in size. In another embodiment, the incision can be no greater than approximately 2.85 mm in size. In another embodiment, the incision is no greater than approximately 2.85 mm and can be greater than approximately 1.5 mm.

After insertion through the incision, the guidewire 515 can be advanced into the anterior chamber along a pathway that enables the implant 105 to be delivered to a position such that the implant 105 provides a flow passageway from the anterior chamber toward the suprachoroidal space. The guidewire 515 can be advanced further into the eye such that the blunt distal tip of the guidewire 515 and/or the implant 105 seats with and can penetrate the iris root IR or a region of the ciliary body CB or the iris root part of the ciliary body near its tissue border with the scleral spur.

The guidewire 515 can approach the iris root from the same side of the anterior chamber as the deployment location such that the guidewire 515 does not have to be advanced across the iris. Alternately, the guidewire 515 can approach the location from across the anterior chamber such that the guidewire 515 is advanced across the iris and/or the anterior chamber toward the opposite iris root. The guidewire 515 can approach the eye and the iris root along a variety of pathways. For example, the guidewire 515 can be advanced through the anterior chamber such that it does not intersect the optical axis of the eye. In other words, the corneal incision and the location where the implant 105 is implanted at the iris root can be in the same quadrant (if the eye is viewed from the front and divided into four quadrants).

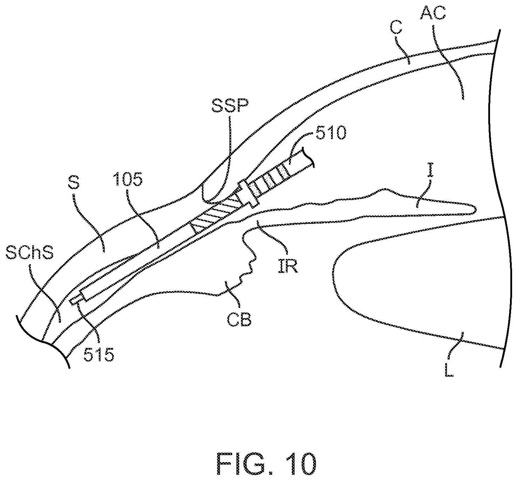

FIG. 10 shows an enlarged view of the anterior region of the eye showing the anterior chamber AC, the cornea C, the iris I, and the sclera S. In addition, FIG. 10 shows the implant 105 loaded onto a guidewire 515 and approaching the supraciliary space or suprachoroidal space from the anterior chamber AC. The implant 105 mounted on the guidewire 515 can move along a pathway such that the dissection entry point of the distal tip of the guidewire 515 can penetrate the iris root IR near its junction with the scleral spur SSp or the iris root portion of the ciliary body CB or other desired location. The surgeon can rotate or reposition the handle 310 of the delivery system 305 in order to obtain a proper approach trajectory for the distal tip of the guidewire 515, as described in further detail below.

The guidewire 515 with the implant 105 positioned thereupon can be advanced from a region of the anterior chamber which can be viewed through a transparent zone of the cornea to a region of the anterior chamber that may be obscured by an opaque zone of the cornea. The guidewire 515 and implant 105 can be advanced through the cornea C until resistance is felt and the delivery device can be seated at a location near the iris root IR, the ciliary body or the iris root portion of the ciliary body. The guidewire 515 can then be advanced further such that the guidewire 515 and implant 105 loaded thereon can penetrate an area of fibrous attachment between the scleral spur SSP and the ciliary body CB. This area of fibrous attachment can be approximately 1 mm in length. Once the distal tip of the guidewire 515 penetrates and is urged past this fibrous attachment region, the guidewire 515 can then more easily cause the sclera S to peel away or otherwise separate from the ciliary body CB and possibly the choroid as the guidewire 515 follows the inner curve of the sclera S and enters the supraciliary space. A combination of the guidewire's tip shape, material, material properties, diameter, flexibility, compliance, coatings, pre-curvature etc. can make it more inclined to follow an implantation pathway which mirrors the curvature of the inner wall of the sclera and between tissue layers such as between the sclera and the ciliary body, and between the sclera and the choroid.

The dissection plane of the guidewire 515 and implant 105 can follow the curve of the inner scleral wall such that the implant 105 mounted on the guidewire 515 can bluntly dissect the boundary between the scleral spur SSp and the ciliary body CB such that a distal region of the implant extends into the supraciliary space. For example, the dissection plane can be formed by the guidewire 515 and implant 105 after either the guidewire 515 or implant 105 penetrates the iris root or the iris root portion of the ciliary body. The implant 105 can be positioned such that it does not extend anteriorly past the scleral spur SSP far enough to reach or otherwise contact the choroid. In addition, in some embodiments, the distal end of the implant 105 does not reach and cannot contact the choroid. In another embodiment, the implant 105 can extend sufficiently past the scleral spur SSP such that it can be positioned between the tissue boundaries of the sclera and the choroid (the suprachoroidal space).

In some examples at least approximately 1 mm to approximately 2 mm of the implant (along the length) remains in the anterior chamber AC. The implant 105 can be positioned so that a portion of the implant 105 is sitting on top of the ciliary body CB. The ciliary body CB may act as a platform off of which the implant 105 can cantilever towards or into the suprachoroidal space SChS although the implant may not actually enter the suprachoroidal space. The implant 105 can lift or "tent" the sclera S outward such that a tented chamber is formed around the distal end of the implant 105. It should be appreciated that the actual contour of the tented region of tissue may differ in the actual anatomy. In some examples the distal end of the implant 105 does not extend far enough to reach the choroid. In another embodiment, the distal end of the implant 105 reaches the choroid and can contact the choroid.

Once properly positioned, the implant 105 can then be released from the guidewire 515. The implant 105 can be released for example by withdrawing the guidewire 515 such that the implant 105 is effectively disengaged in a controlled manner from the tip of the guidewire 515 with the assistance of the sheath 510, as described above.

The implant 105 can include one or more structural features near its proximal region that aid to anchor or retain the implant 105 in the target location in the eye. The structural features can include flanges, protrusions, wings, tines, or prongs, and the like which can lodge into surrounding eye anatomy in order retain the implant 105 in place and prevent the implant 105 from moving further into the suprachoroidal space SchS.

The delivery system 305 can be used in combination with any number of devices and systems in order to complete a variety of procedures. For example, the delivery system 305 can be used with a direct visualization (DV) system which is configured and adapted to measure one or more anatomical features of the eye, including the iridocorneal angle of the eye. The DV system can, for example, provide a user with measurements which can allow the user to determine an appropriately sized implant for implantation into the eye. In addition, the measurements can assist the user in properly positioning and implanting the implant into the eye, including the implant 105 described above. More specifically, the delivery system 305 can utilize the one or more measurements taken by the DV system in order to assist the delivery system 305 in properly positioning and implanting an appropriately sized implant.

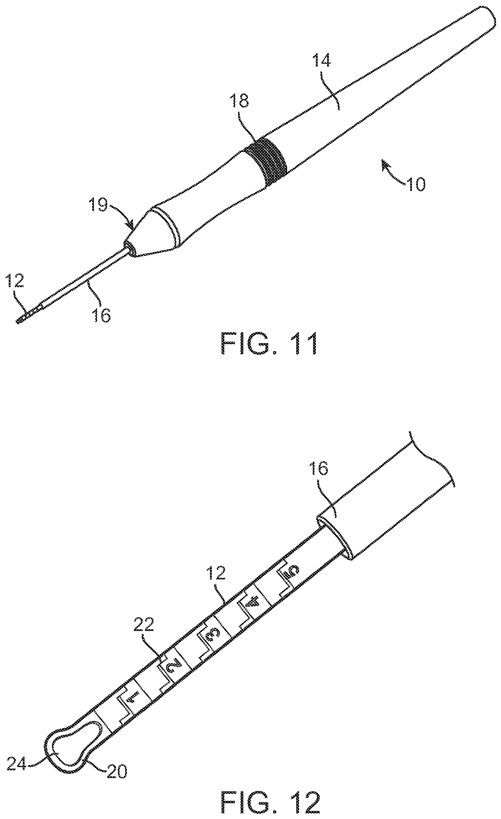

FIG. 11 shows a perspective view of an embodiment of a DV system 10 which can be comprised of a hand-held tool having a DV wire 12 that is movably coupled to an elongated handle 14. At least a portion of the DV wire 12 can be slidably and axially-positioned in a stopper tube 16 affixed to a distal end 19 of the handle 14. Both the stopper tube 16 and the DV wire 12 can extend outward from the distal end 19 of the handle 14. The handle 14 can be sized and shaped to be held in a single hand of a user. In addition, the handle 14 can be configured such that the DV system 10 can be operated single handedly. Furthermore, the handle 14 can have one or more gripping features 18, such as ridges and cutouts, for improved ergonomics and ease of holding.

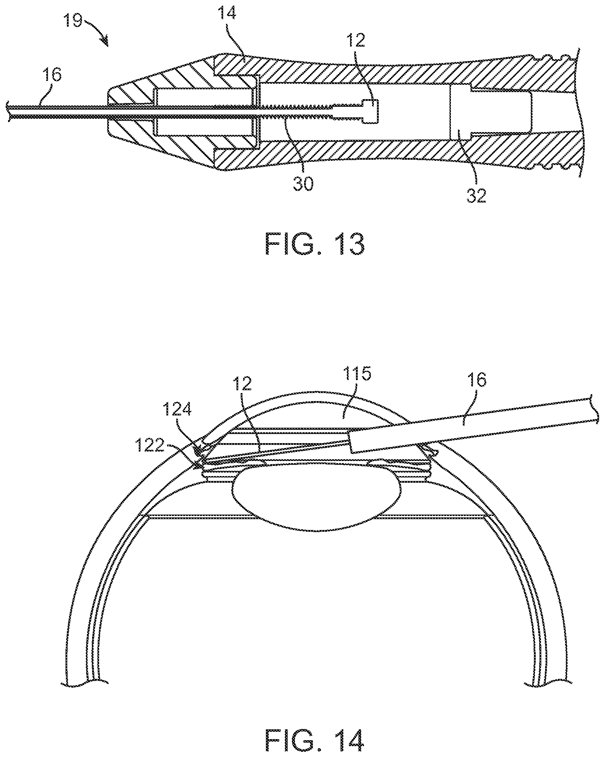

The DV wire 12 can be coupled to a spring 30 inside the handle 14 which can allow the DV wire 12 to move inward and outward along a longitudinal axis of the DV system 10 and relative to the handle 14 and stopper tube 16. The spring 30 can provide a spring force that can assist in allowing the DV wire 12 to retract proximally into the handle 14 upon an applied force against the distal end of the DV wire 12. The spring constant of the spring 30 can be relatively low such that the DV wire 12 moves relatively easily when a force is applied. In one aspect, the spring constant can be sufficiently low such that the DV wire 12 will yield and ocular tissue is not damaged when the distal tip of the DV wire 12 is pressed against ocular tissue. In addition, a handle plug 32 (as shown in FIG. 13) inside the handle 14 can provide a hard stop for the DV wire 12 which can limit the distance that the DV wire 12 can retract into the stopper tube 16 and handle 14.

In some embodiments the stopper tube 16 can extend straight out of and along the same longitudinal axis as the handle 14. However, in some embodiments the stopper tube 16 can be curved or extend in a variety of other configurations. For example, the stopper tube 16 may be curved which can provide easier access to particular anatomical parts of the eye, such as the base of the iridocorneal angle. The distal end of the stopper tube 16 can have rounded edges which can assist in preventing damage to ocular tissue during use. In addition, the stopper tube 16 can be manufactured out of a variety of materials, such as stainless steel, titanium, plastics, or other equivalent materials, including any number of medical grade materials.

FIG. 12 shows an enlarged view of the DV wire 12 and distal region of the stopper tube 16. The DV wire 12 can have a distal contact tip 20 that can be configured to be pressed against ocular tissue. The contact tip 20 may be rounded or blunt to eliminate or reduce tissue damage by the contact tip 20 when pressed against ocular tissue. In addition, one or more indicators or marks 22 can be positioned along a length of the DV wire 12. In some embodiments, one or more indicators 22 can be positioned along a length of either the DV wire 12 or stopper tube 16. The indicators 22 can assist a user in acquiring measurements of one or more anatomical features of the eye. For example, the distal end of the DV wire 12 can be placed against the base of the angle of the eye such that the user can then determine the depth of the angle.

The indicators 22 can be arranged along the DV wire 12 such that they correspond to a standard form of measurement, i.e., millimeters, fractions of 2.54 cm (an inch), etc. In such an embodiment, a user can use the DV wire 12 to make specific measurements, including measurements of particular anatomical features of the eye. In some embodiments, the indicators 22 do not correlate with a standard form of measurement and are simply reference points along the DV wire 12. In either embodiment, a user can position the DV wire 12 in the eye and use any of the indicators 22 as reference points relative to various anatomical features in the eye. As will be discussed in greater detail below, the referenced indicators 22 can assist the user in subsequent procedures, including determining an appropriately sized implant for the eye as well as assisting in correctly inserting the implant into the eye, including with the delivery system 305.

The DV wire 12 can be manufactured out of a variety of materials, such as stainless steel, titanium, plastics, or other equivalent materials, including any number of medical grade materials. In addition, the DV wire 12 can be at least partially tubular or hollow in order to allow one or more components, such as the measuring features discussed below, to be contained within the DV wire 12, including within the contact tip 20.

The contact tip 20 can be configured to provide sufficient surface area so as to not be traumatic to ocular tissues and/or create accidental cyclodialysis. The indicators 22 can be visible to the physician through the cornea when the DV wire 12 is extended from the stopper tube 16. In addition, the indicators 22 can be visible through the cornea so that a gonio lens is not needed in order to determine the depth of the iridocorneal angle. Furthermore, the DV system 10 can perform sufficient measurements such that a gonio lens is not necessary to perform a procedure. By relieving the need for a gonio lens to conduct a procedure, both procedure time and efficiency can be improved.

The DV wire 12 can be stamped, chemically etched, or marked with any number of patterning techniques in order to provide indicators 22 that can be seen by a user while inserted in the eye. The indicators 22 may exhibit any combination of numbering and or patterning features, with varying degrees of darkness, contrast, size, shape and color.

In some embodiments, the contact tip 20 can include a loop 24 which can provide additional damping when the contact tip 20 is in contact with ocular tissue. In addition, the contact tip 20 can be made out of a material that allows the loop 24 to deform, such as a soft or flexible material, in order to provide a damping effect. The loop 24 can be made out of the same or different material than the rest of the DV wire 12, or the loop 24 can be coated with a material, such as a flexible or soft material.

In some embodiments, deformation of the contact tip 20 or loop 24 can assist in providing a visual cue to the user that the distal end of the DV wire 12, such as the contact tip 20 or loop 24, is in contact with tissue. For example, the contact tip 20 can include one or more features having a spiral cut or any number of a variety of looped patterns which can allow for visually identifiable movements at low forces. Furthermore, deformation of the loop 24 can act as a deformable element which can provide visual cues to the user, such as when the loop 24 is in contact with tissue.

The cross section of the DV wire 12 can be rectangular, although the shape may vary. For example, the DV wire 12 can have a circular, elliptical or any one or more of a variety of cross sections along the length of the DV wire 12. In addition, the edges of the DV wire 12 can be smooth and free of sharp edges to avoid damage to tissue. The proximal end of the DV wire 12 can have ridges for holding the spring 30 in place as well as a hard stop to prevent the spring 30 from sliding off the proximal end.

FIG. 13 shows a cross-sectional view of a part of the DV system 10, including the distal end 19 of the handle 14. The DV wire 12 of the DV system 10 can be coupled to a spring 30 at a proximal region which can bias the DV wire 12 toward a distally outward direction relative to the handle 14. In addition, the spring 30 can resist movement of the DV wire 12 in a proximal direction (i.e., into the handle 14) and urge the DV wire in a distal direction (i.e., out of the handle 14).

The spring 30 can be a low force spring (i.e., a spring constant in the range of .001 to .100 Newtons). The spring 30 may be made of Nitinol, stainless steel, titanium, plastics, or other equivalent materials, and may exhibit strain induced deformation. Additionally, the spring 30 may be at least one of a tension spring, compression spring, torsion spring, leaf spring, Belleville washer, constant force spring, or urethane spring. The spring 30 may be an ultra-low force spring (i.e., less than .001 Newtons) for greater sensitivity, or a higher force spring (i.e., greater than .100 Newtons) for overcoming frictional viscous forces of aqueous fluids.

One or more features may be added or removed from the DV system 10 based on its intended use (i.e., disposable, re-usable, etc.). For example, one or more holes through the handle 14 and handle plug 32 may be included in the system in order to allow for sterilization and re-use of the DV system 10. Other features can be implemented for special or improved use of the DV system 10.

FIG. 14 shows an example of a part of the distal region of the DV system 10 inserted in an eye. The DV system 10 can be inserted into the anterior chamber 115 of the eye via a corneal or limbal incision such that the DV wire 12 can pass across the anterior chamber 15 (pursuant to an ab-interno approach) toward the base of the angle, such as below the scleral spur 124 and above the iris 122. The distal end of the DV wire 12, such as the contact tip 20, can be pressed against ocular tissue, as shown by way of example in FIG. 14.

The DV wire 12 can apply a force against ocular tissue while the handle 14 and stopper tube 16 continue to advance in the direction of the eye. The spring 30 can allow the proximal end of the DV wire 20 to travel towards the handle plug 32 while the handle 14 and stopper tube 16 continue to travel in the direction of the eye. In some embodiments, the DV wire 20 can continue to retract into the handle 14 until the proximal end of the DV wire 20 abuts the handle plug 32. Retraction of the DV wire 20 into the stopper tube 16 and handle 14 can indicate to the user that the contact tip 20 of the DV wire 12 is properly positioned, such as the distal end of the DV wire 12 is positioned against the base of the angle. This can assist in at least minimizing damage to the ocular tissue by preventing the user from applying more force than is necessary when attempting to properly position the DV wire 12 in the eye.

Once the surgeon becomes aware that the DV wire 20 is properly positioned, the surgeon can then take appropriate measurements, such as of the iridocorneal angle of the eye. Measurements can be made by, for example, referencing the indicators 22 along the DV wire 12 relative to one or more anatomical features of the eye. After measurements have been taken, the surgeon can then retract the distal end of the DV system 10 from the eye. Any number of procedures can follow the removal of the DV system 10, including the insertion of an ocular implant, such as with the delivery system 305 described above.

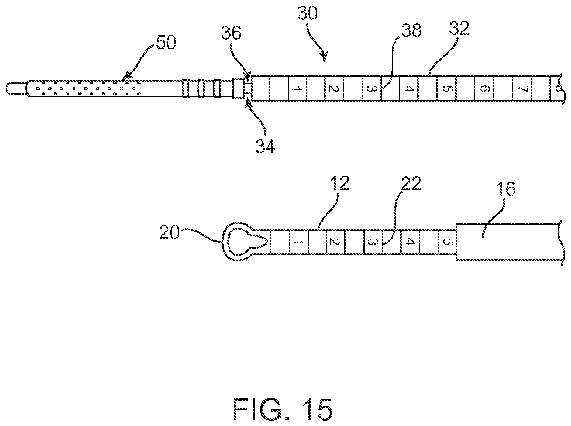

FIG. 15 shows the distal end of the DV system 20, including the DV wire 12, aligned alongside a distal end of an implant delivery applier 30. The implant delivery applier 30 can have an elongated body 32 with an adaption feature 34 at a distal end 36 of the elongated body 32. The adaptation feature 34 can be configured to adapt one or more ocular implants 50 to the distal end 36 of the implant delivery applier 30, as shown in FIG. 15. The body 32 of the implant delivery applier 30 can include indicators or marks 38 which correspond with the indicators 22 along the DV wire 12, as also shown in FIG. 15. The corresponding indicators along the implant delivery applier 30 and DV wire 12 can allow measurements and positioning of the DV wire 12 relative to anatomical features of the eye to be easily replicated with the implant delivery applier 30, as will be discussed in greater detail below.

In addition, the delivery system 305 described above can include one or more features of the implant delivery applier 30 such that the delivery system 305 can be used similarly to the implant delivery applier 30 as described herein. For example, the delivery system 305 can include one or more indicators or marks which correspond with the one or more indicators 22 along the length of the DV wire 12. However, any function or feature disclosed or suggested herein relating to the implant delivery applier 30 can be included in the delivery system 305. Similarly, any function or feature disclosed or suggested herein relating to the delivery system 305 can be included in the implant delivery applier 30.

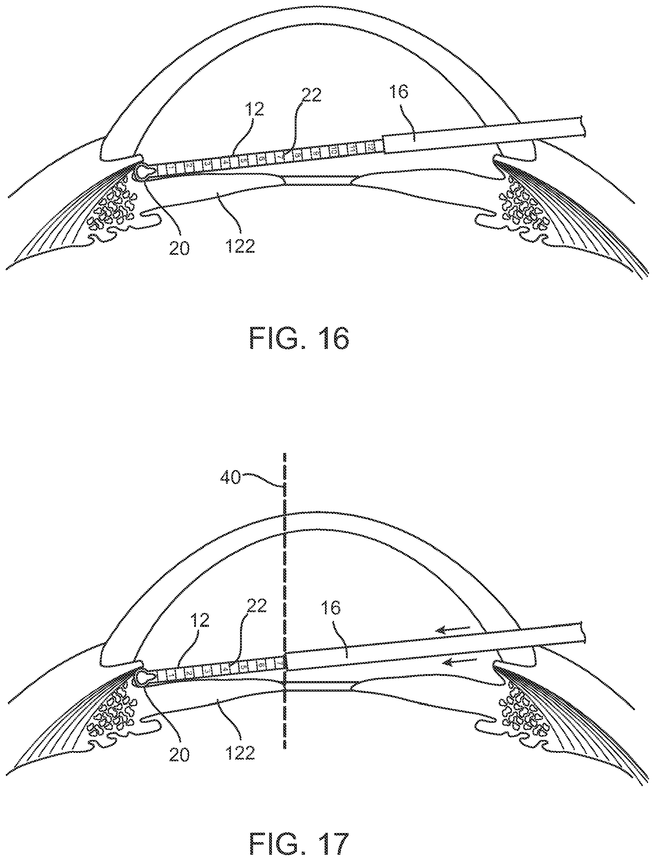

FIGS. 16-19 show an example method of use of the implant delivery applier 30 and DV wire 12 of the DV system 10 having corresponding marks 38 and 22, respectively, for properly inserting an implant in the eye. The method shown can be used, for example, to at least acquire one or more measurements of the eye, determine a properly sized implant and implant the properly sized implant, such as the implant 105 described above, into the eye. Furthermore, this method can be completed without the use of a gonio lens which can improve the time and efficiency of the procedure.

As shown in FIG. 16, a user can first insert the distal end of the DV wire 12 through a corneal or limbal incision along the eye and advance the distal end of the DV wire 12 across the anterior chamber of the eye (pursuant to an ab-interno approach). Viscoelastic substances or balanced saline solutions may be used to maintain the anterior chamber of the eye and open a space comprising a part of the angle of the eye. The incision can be approximately .08mm to 2.0mm in length and can be either created by the DV wire 12 or a separate instrument. Additionally, the incision can be approximately 1.2mm to 1.7mm in length.

The user can advance the DV system 10 and position the distal end of the DV wire 12 against ocular tissue, such as between the scleral spur 124 and iris 122 in order to measure the depth of the iridocorneal angle. The spring loaded feature of the DV wire 12 can assist the user in determining when the distal end, such as the loop 24 or contact tip 20, of the DV wire 12 is in contact with ocular tissue. For example, the user can continue to advance the DV system 10 into the eye until the user begins to observe the stopper tube 16 travel over the DV wire 12. Movement of the stopper tube 16 relative to the DV wire 12 can alert the user that the distal end of the DV wire 12 is positioned against ocular tissue within the eye.

Once the user has determined that the distal end of the DV wire 12 is positioned against the base of the angle of the eye, such as between the scleral spur 124 and iris 122, the user can take measurements of the eye using the DV wire 12. For example, the user can use the indicators 22 along the DV wire 12 to take measurements of certain anatomical features of the eye, including the depth of the angle of the eye. As shown in FIG. 17, the user can view the DV wire 12 along a generally vertical line of sight 40 in order to observe which indicator 22 is aligned with one or more anatomical features of the eye when the distal end of the DV wire 12 is positioned against the base of the angle. For example, the user can view the DV wire 12 along the vertical line of sight 40 and observe which indicator 22 is aligned with, for example, the inner edge of the iris 122. Any number of anatomical features can be measured using the indicators 22 along the DV wire 12 without departing from the scope of this disclosure.

In addition, the user can advance a feature of the DV system 10, such as the stopper tube 16, in order to assist the user in determining which indicator 22 is aligned with certain anatomical features of the eye. FIG. 17 shows an example of the stopper tube 16 being used to assist the user in determining which indicator 22 or part of the DV wire 12 aligns with the inner edge of the iris 122 when the distal end of the DV wire 12 is placed against the base of the iridocorneal angle in order to measure the depth of the angle. The stopper tube 16 can be advanced across the DV wire 12 by simply continuing to advance the DV system 10 after the distal end of the DV wire 12 is positioned against ocular tissue within the angle of the eye, as discussed above.

Once the user has obtained appropriate measurements, the user can remove the DV wire 12 from the eye. The implant 50 coupled to the implant delivery applier 30, or delivery system 305, can then be inserted into the eye. The same incision that was used to insert the DV wire 12 can be used to insert the implant delivery applier 30 and implant 50. In addition, the implant 50 can be advanced across the eye along the same or similar trajectory such that the distal end of the implant 50 contacts generally the same area of ocular tissue between the scleral spur 124 and iris 122 that the distal end of the DV wire 12 had previously contacted while taking measurements.

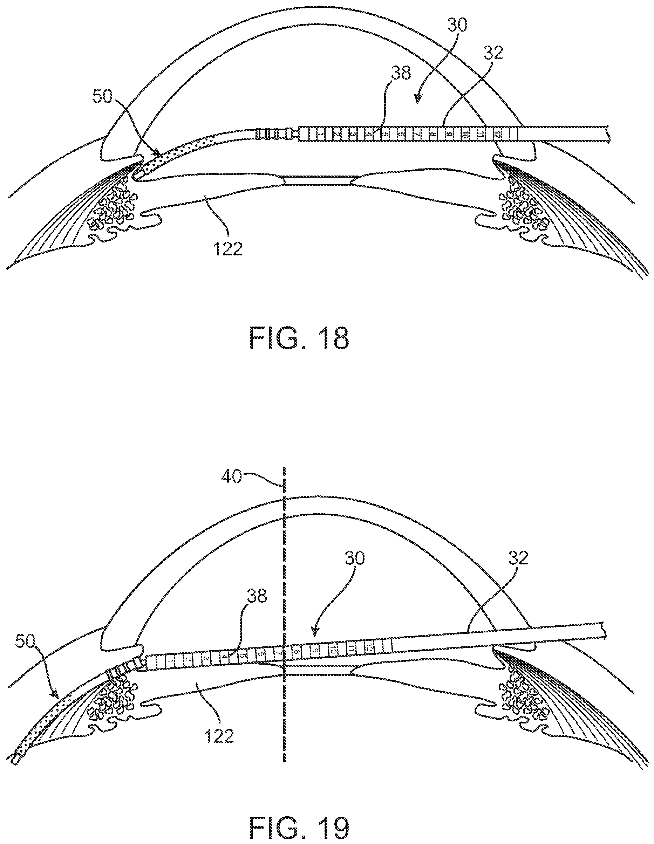

As shown in FIGS. 18 and 19, the implant delivery applier 30 can be advanced in order to allow the implant 50 to be inserted into the suprachoroidal or supraciliary space. The user can continue to advance the implant 50 into the suprachoroidal or supraciliary space until one or more indicator 38 along the implant delivery applier 30 aligns with one or more anatomical features of the eye. In particular, the user can advance the implant delivery applier 30 until the same indicator 38 along the implant delivery applier 30 is aligned with the iris 122 as was along the DV wire 12 when the distal end of the DV wire 12 was in contact with the base of the angle (see, for example, FIGS. 17 and 19).

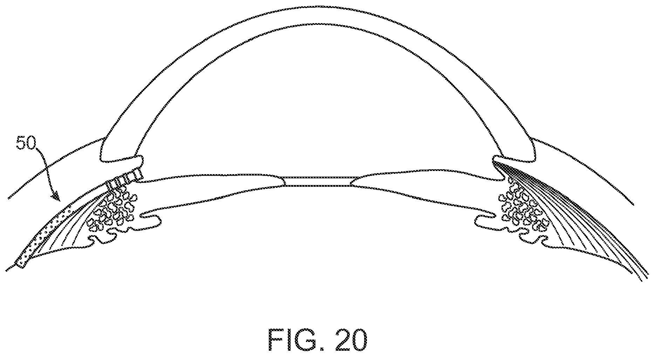

As shown in FIG. 19, the user can advance the implant delivery applier 30 until the user observes a particular anatomical feature of the eye align with an indicator 38 along the implant delivery applier 30 which corresponds to an indicator 22 along the DV wire 12 which had previously been aligned with the same particular anatomical feature, such as when the distal end of the DV wire 12 was in contact with the base of the angle. When this corresponding indicator 38 on the implant delivery applier 30 is aligned with the particular anatomical feature of the eye, the user can determine that the implant 50 is properly positioned in the eye for permanent implantation. For example, proper positioning in the eye for permanent implantation includes positioning the implant so that it can provide fluid communication between the anterior chamber and the suprachoroidal or supraciliary space without discomfort or irritation to the eye. Therefore, once the user has aligned the appropriate indicator 38 along the implant delivery applier 30 with the particular anatomical feature, the user can then release the implant 50 from the implant delivery applier 30 and remove the implant delivery applier 30 from the eye. As shown in FIG. 20, the implant 50 can then remain in the implanted position permanently or for a desired length of time.

The DV wire can be aligned with the implant delivery applier such that the distal end of the DV wire aligns with a position along the length of the head of the implant 50 when the implant 50 is coupled to the implant delivery applier 30. The alignment of the distal end of the DV wire 12 relative to the head of the implant 50 coupled to the implant delivery applier 30 can vary depending on the desired placement of the head relative to the anterior chamber of the eye when the implant 50 is in its permanently implanted position. For example, and shown by way of example in FIG. 20, it may be beneficial to have at least a portion of the head of the implant 50 extend into the anterior chamber of the eye. This can assist in ensuring that the implant 50 provides a fluid pathway between the anterior chamber and supraciliary or suprachoroidal space.

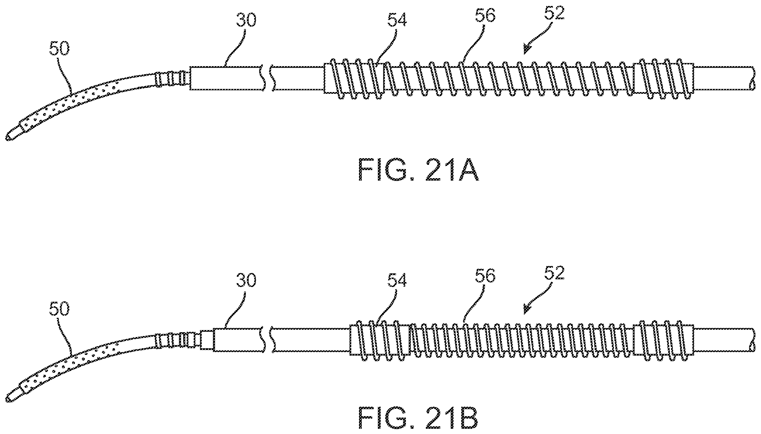

FIGS. 21A-21B shows an embodiment of a feedback mechanism 52 coupled to or comprising the implant delivery applier 30. The feedback mechanism 52 can include a sheath 54 coupled to a spring 56 at a proximal end of sheath 52. In such an embodiment, the spring loaded sheath 54 can be used to indicate depth or acknowledge when a certain landmark has been reached. For example, the sheath 54 can be positioned such that the distal end of the sheath 54 extends a distance over the implant 50 attached to the distal end of the implant delivery applier 30. Upon implantation of the implant 50 within the eye, the sheath 54 can be pushed in the proximal direction, or retracted, when the implant 54 has been implanted to a preferred depth within the eye. Retraction of the sheath 54 can indicate to a user that the sheath 54 has hit a hard stop, such as ocular tissue, and the implant 50 has been properly implanted. The implant 50 can then be released for permanent implantation once proper implant positioning has been determined.

In addition, the feedback mechanism 52 can assist the user in positioning the implant 50 such that the proximal end of the implant 50 is in direct communication with the anterior chamber of the eye in an implanted state. This can ensure that the implant 50 can provide a fluid path from the anterior chamber of the eye to another part of the eye, such as to the suprachoroidal or supraciliary space, and improve fluid flow within the eye.

Furthermore, the DV system 10 can be used for a variety of surgical procedures. For example, the DV system can be used to accurately locate and take measurements relating to a variety of anatomical structures, such as the trabecular meshwork and the Schlemm's Canal. The various measurements taken with the DV system 10 can be used for accurately positioning implants into one or more anatomical structures, including at least the trabecular meshwork and Schlemm's Canal.

Furthermore, in some embodiments, the distal end of the DV system 10, such as the distal end of the DV wire 12, can include noncontact measuring features for determining one or more of a measurement or a distance within the eye. For example, the distal end of the DV wire 12 can include one or more measuring features which can include ultrasound, infrared, optical coherence tomography, or the like. In some embodiments, the measuring features can assist in measuring the relative distance of an anatomical feature of the eye relative to a part of the DV wire 12, such as the distal end. Additionally, the DV wire 12 can include various other features which can assist in providing information to a user, such as pressure and temperature sensors.

In some embodiments, the handle can include a display which can indicate to a user one or more parameters measured by the DV system 10, such as by a measuring feature of the DV system 10. Information displayed on the display can include, for example, at least one or more of a distance measured between the distal end of the DV wire 12 and an anatomical feature, a measurement of an anatomical feature, a pressure exerted by the distal end of the DV wire 12 against tissue, pressure within the eye or temperature.

At least some optical implants are small, such as having lengths and widths on the order of millimeters, which can make it difficult for a user to manipulate the implant. In particular, it can be difficult for the user to prepare the implant for loading as well as loading the implant onto a delivery device, such as the implant delivery applier 30 and delivery system 305 described above. Therefore, it can be beneficial to have a device which can assist in protecting the implant, including the implants 105 and 50 disclosed herein, prior to loading onto a delivery device. In addition, it can be beneficial to have a device which can assist in loading the implant onto the delivery device. Furthermore, it can be beneficial to have a device which assists in ensuring that the implant is properly loaded onto the delivery device without damaging the implant.

The present disclosure includes a pencap implant loader which can assist in protecting the implant, including during storage and loading the implant onto a delivery device. In addition, the pencap implant loader can assist in loading the implant onto the delivery device and ensure that the implant is properly loaded onto the delivery device without damaging the implant. Therefore, the pencap implant loader embodiments disclosed herein can improve surgery time, streamline surgery procedures, and at least minimize implant loading related complications.

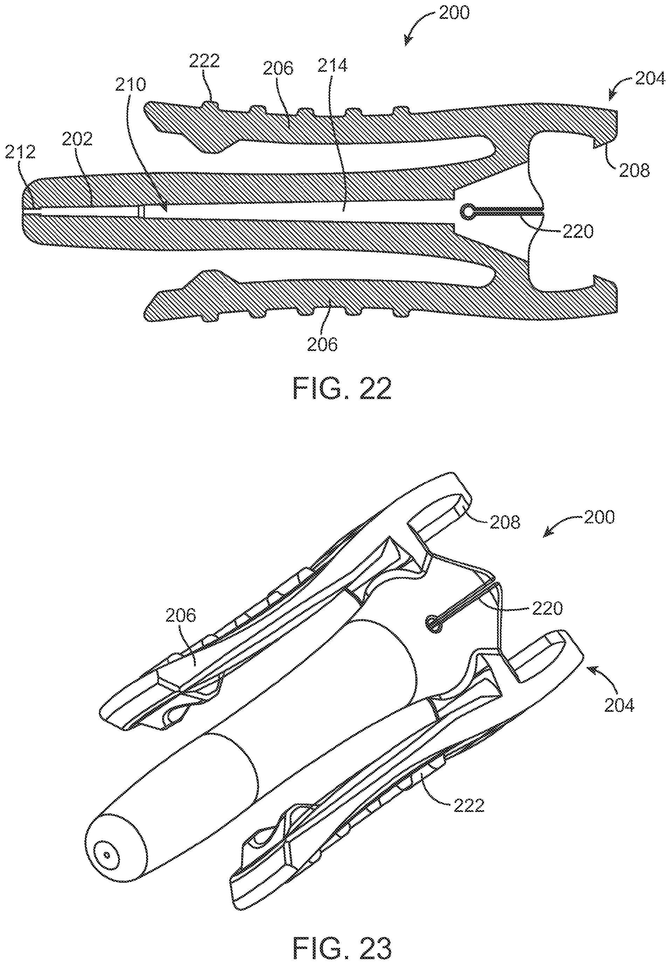

FIGS. 22 and 23 illustrate an embodiment of a pancap implant loader 200 which includes an implant housing 202 and a delivery device adapter 204. The implant housing 202 can be configured to house an implant at least either prior to or during loading of the implant onto the delivery device. In addition, the implant housing 202 can be configured to house a variety of one or more implants, including the implants 105 and 50 disclosed herein.

The delivery device adapter 204 can be configured to adapt to any number of implant delivery devices, including the implant delivery applier 30 and delivery system 305 described above. In some embodiments, the delivery device adapter 204 can include a pair of retention arms 206 which can have retention features 208 which can grasp and secure the delivery device in a position relative to the pencap implant loader 200. For example, the retention arms 206 and retention features 208 can secure the delivery device relative to the pencap implant loader 200 such that the delivery device can effectively and efficiently load the implant contained in the implant housing 202 onto the delivery device. Furthermore, the retention arms 206 and retention features 208 can secure the delivery device such that an implant loading feature of the delivery device is aligned with the implant housing 202 in order to allow the implant loading feature, such as a guidewire, to load the implant correctly and without damage to the implant.

In some embodiments, the delivery device adapter 204 can include at least one spring loaded retention arm 206, as shown in FIG. 22. The spring loaded retention arm 206 can allow a user to squeeze the retention arms 206 in order to couple or decouple the delivery device from the pencap implant loader 200. In addition, the retention features 208 can securely mate with features along the delivery device in order to secure the coupling between the pencap implant loader 200 and the delivery device.

The retention arms 206 can be made out of a variety of materials, including materials that provide the retention arms 206 with spring-loading for coupling and decoupling the delivery device. However, the retention arms 206 can be made out of one or more of a variety of materials. In addition, the retention arms 206 can include one or more gripping features 222, including ridges along a length of the handle. The gripping features 222 can assist a user in grasping and manipulating the pencap implant loader 200.

In some embodiments, the pencap implant loader 200 can include a relief 220 which can assist in allowing a distal end of the delivery device to mate with the pencap implant loader, such as without becoming jammed in the pencap implant loader 200. In addition, the relief 220 can assist in allowing the retention arms 206 to couple and decouple the delivery device, such as by allowing additional movement of the retention arms 206.

The pencap implant loader 200 can be made out of any number of a variety of materials, including stainless steel, titanium, plastics, or any medical grade or similar materials. In addition, the pencap implant loader 200 can include a passageway 210 which extends through at least a part of the pencap implant loader 200. The passageway 210 can allow the implant to load into the implant housing 202 as well as allow a guidewire or other component of the delivery device to advance into the pencap implant loader 200 in order to load the implant onto the delivery device.

In some embodiments, the passageway 210 can include more than one inner diameter. For example, a distal segment 212 of the passageway can have the smallest diameter along the length of the passageway 210 which can be sized and shaped to allow a guidewire to pass through. In addition, the implant housing 202 can comprise a middle second segment of the passageway 210 which can be sized and shaped to allow the implant and guidewire to be inserted. However, the distal segment 212 can be sized and shaped to only allow the guidewire to pass through and prevent the implant from passing through. This can ensure that the implant is properly contained within the implant housing 202 and cannot travel more distal than the implant housing 202.

In addition, the passageway 210 can include a proximal third segment 214 which can have a larger diameter than either the distal segment 212 or the implant housing 202 in order to allow at least the implant and guidewire to pass through. The third segment 214 can also be sized and shaped to allow a distal part of the delivery device to insert at least a distance into the third segment 214. For example, the third segment 214 can allow at least a part of the stopper tube 510 of the implant delivery applier 30 to insert a distance within the third segment 214.

As will be discussed below, some embodiments of the pencap implant loader 200 can be configured to be coupled to the delivery device, such as the implant delivery applier 30, including during storage of the pencap implant loader 200 and delivery device. Therefore, at least one segment of the passageway can be configured to allow at least a part of the delivery device to couple to the pencap implant loader 200 in order to allow the delivery device to releasably couple to the pencap implant loader 200 for an extended period of time.

An example method of use of the pencap implant loader 200 includes coupling the pencap implant loader 200 having at least one implant contained in the implant housing 202 to the delivery device. The coupled configuration of the pencap loader 200 to the delivery device is then packaged and stored for later use by a user. Upon use, the user can remove the coupled configuration of the pencap implant loader 200 and the delivery device from the packaging and decouple the pencap implant loader 200 from the delivery device. Upon decoupling, the delivery device includes at least one implant that was housed in the implant housing loaded onto the delivery device for implantation into an eye.

In some embodiments, prior to decoupling the pencap implant loader 200 from the delivery device, the user can cause an implant delivery feature, such as a guidewire, to extend into the implant housing 202 in order to load the at least one implant onto the implant delivery feature. Once the implant delivery feature has sufficiently advanced into the implant housing such that the implant is loaded onto the implant delivery feature, the pencap implant loader 200 can be decoupled from the delivery device.

In some embodiments, the pencap implant loader 200 can be loaded with one or more implants and stored prior to use without being coupled to a delivery device. Therefore, upon use, the user can manually couple the pencap implant loader 200 to the delivery device in order to load the at least one implant contained in the pencap implant loader 200 onto the delivery device.

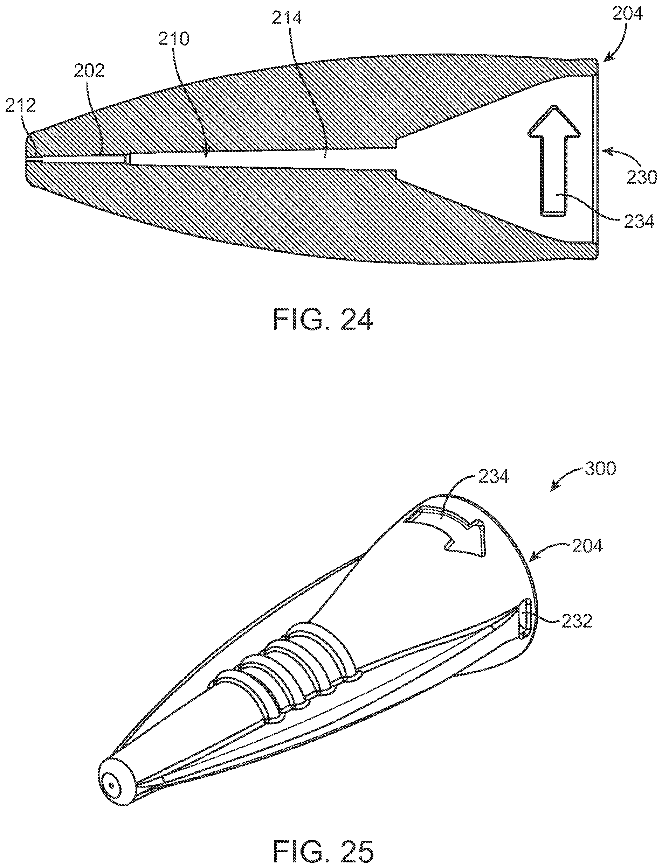

FIGS. 24 and 25 illustrate another embodiment of the pencap implant loader 300 including an implant housing 202 and a delivery device adapter 204. The pencap implant loader 300 can include one or more of the same features as discussed above with regards to the pencap implant loader 200, including a distal first segment 212 and a proximal third segment 214. In addition, the delivery device adapter 204 includes a twisting retention feature 230 which provides a similar function as the retention arms 206 discussed above.

The twisting retention feature 230 can include at least one retaining pin feature 232 which can assist in coupling the pencap implant loader 300 to the delivery device. In some embodiments, in order to decouple the pencap implant loader 300 from the delivery device, the user can twist the pencap implant loader 300, such as in the direction of the arrow 234 shown in FIGS. 24 and 25. Upon twisting of the pencap implant loader 300, the retaining pin feature 232 can assist in relieving the twisting retention feature from securing the coupling between the pencap implant loader 300 and the delivery device.

In some embodiments, the pencap implant loader 200 and 300 can include a feature that either prevents or allows re-capping of the pencap implant loader onto the delivery device. In addition, the pencap implant loader 200 and 300 can include a springing feature which can bias the implant proximally and bias the implant towards a correct position during loading.

## Claims

1. A delivery device (305) for delivering an ocular implant (105) into an eye, the delivery device comprising:
a proximal handle portion (310);
a distal delivery portion (312) coupled to a distal end of the handle portion (310) and configured to releasably hold an ocular implant (105), the ocular implant (105) being an elongate element having one or more internal lumens, the delivery portion (312) comprising a sheath (510) positioned axially over a guidewire (515), wherein the guidewire (515) includes at least one retention feature along a length of the guidewire (515) which assists in retaining the ocular implant (105) along the length of the guidewire (515), **characterized in that** the at least one retention feature includes a sinusoidal configuration along a length of the guidewire (515), the sinusoidal configuration comprising at least one curved region (524) that is configured to provide an interference fit between the guidewire (515) and an inner lumen of the ocular implant (105);
an actuator (420) coupled to a mechanism that releases the ocular implant (105) from the delivery portion (312) upon actuation of the actuator (420), wherein the guidewire (515) is coupled to a part of the mechanism which includes a spring (550), wherein the mechanism is configured to retract the guidewire (515) toward the sheath upon actuation of the actuator (420); and
a dampener (565) coupled to the handle portion (310), the dampener (565) adapted to dampen retraction of the guidewire (515).

2. The delivery device (305) of claim 1, wherein a distal end of the guidewire (515) extends distally from a distal end of the sheath (510) when the spring (550) is in a first formation.

3. The delivery device (305) of claim 2, wherein the distal end of the guidewire (515) proximally retracts from the distal end of the sheath (510) when the spring (550) is in a second formation.

4. The delivery device (305) of claim 3, wherein actuation of the actuator (420) causes the spring (550) to transition between the first formation and the second formation.

5. The delivery device (305) of claim 1, wherein a distal end of the sheath (510) is at least one of receives or abuts a proximal end of the ocular implant (105) and prevents the ocular implant (105) from sliding in a proximal direction as the guidewire (515) proximally retracts.

6. The delivery device (305) of claim 1, wherein the sinusoidal configuration includes a radius of curvature in the range of 1.0795 cm (0.425 inches) to 1.3335 cm (0.525 inches).

7. The delivery device (305) of claim 1, wherein a peak-to-peak distance between two or more retention features or curved regions (524) along the length of the guidewire (515) is within a range of 0.0254cm (0.0100 inch) to 0.0508 cm (0.0200 inch).

8. The delivery device (305) of claim 1, wherein the guidewire (515) includes at least one inner lumen.

9. The delivery device (305) of claim 8, wherein the guidewire (515) includes at least one opening along a length of the guidewire (515) which provides fluid communication between the inner lumen of the guidewire (515) and an area surrounding the guidewire (515).

10. The delivery device (305) of claim 9, wherein the area surrounding the guidewire (515) is at least one of an inner lumen of the ocular implant (105) or a part of the eye.

11. The delivery device (305) of claim 10, wherein the implant includes at least one hole along a length of the ocular implant (105) which provides fluid communication between the inner lumen of the implant and an area surrounding the ocular implant (105).

12. The delivery device (305) of claim 1, wherein the delivery device (305) includes at least one fluid delivery feature which delivers fluid from a fluid source to at least one of the ocular implant (105) or the eye.

13. The delivery device (305) of claim 12, wherein the fluid from the fluid source includes one or more of a viscoelastic, a drug and a stem cell.

## Patentansprüche

1. Zuführungsvorrichtung (305) zum Zuführen eines okularen Implantats (105) in ein Auge, wobei die Zuführungsvorrichtung Folgendes umfasst:
einen proximalen Griffabschnitt (310);
einen distalen Zuführungsabschnitt (312), der mit einem distalen Ende des Griffabschnitts (310) gekoppelt und zum lösbaren Halten eines okularen Implantats (105) konfiguriert ist, wobei das okulare Implantat (105) ein längliches Element mit einem oder mehreren Innenlumen ist, wobei der Zuführungsabschnitt (312) einen Mantel (510) umfasst, der axial über einem Führungsdraht (515) positioniert ist, wobei der Führungsdraht (515) wenigstens ein Haltemerkmal entlang einer Länge des Führungsdrahts (515) aufweist, das beim Festhalten des okularen Implantats (105) entlang der Länge des Führungsdrahts (515) assistiert, **dadurch gekennzeichnet, dass** das wenigstens eine Haltemerkmal eine sinusförmige Konfiguration entlang einer Länge des Führungsdrahts (515) aufweist, wobei die sinusförmige Konfiguration wenigstens eine gekrümmte Region (524) aufweist, die zum Erzielen einer Presspassung zwischen dem Führungsdraht (515) und einem Innenlumen des okularen Implantats (105) konfiguriert ist;
einen Aktuator (420), der mit einem Mechanismus gekoppelt ist, der das okulare Implantat (105) nach Betätigung des Aktuators (420) vom Zuführungsabschnitt (312) löst, wobei der Führungsdraht (515) mit einem Teil des Mechanismus gekoppelt ist, der eine Feder (550) beinhaltet, wobei der Mechanismus zum Zurückziehen des Führungsdrahts (515) in Richtung der Hülle nach Betätigung des Aktuators (420) konfiguriert ist; und
einen Dämpfer (565), der mit dem Griffabschnitt (310) gekoppelt ist, wobei der Dämpfer (565) zum Dämpfen des Zurückziehens des Führungsdrahts (515) ausgelegt ist.

2. Zuführungsvorrichtung (305) nach Anspruch 1, wobei ein distales Ende des Führungsdrahts (515) distal von einem distalen Ende des Mantels (510) verläuft, wenn die Feder (550) in einer ersten Formation ist.

3. Zuführungsvorrichtung (305) nach Anspruch 2, wobei sich das distale Ende des Führungsdrahts (515) proximal vom distalen Ende des Mantels (510) zurückzieht, wenn die Feder (550) in einer zweiten Formation ist.

4. Zuführungsvorrichtung (305) nach Anspruch 3, wobei das Betätigen des Aktuators (420) bewirkt, dass die Feder (550) zwischen der ersten Formation und der zweiten Formation übergeht.

5. Zuführungsvorrichtung (305) nach Anspruch 1, wobei ein distales Ende des Mantels (510) ein proximales Ende des okularen Implantats (105) aufnimmt oder daran anstößt und verhindert, dass das okulare Implantat (105) in einer proximalen Richtung gleitet, während sich der Führungsdraht (515) proximal zurückzieht.

6. Zuführungsvorrichtung (305) nach Anspruch 1, wobei die sinusförmige Konfiguration einen Krümmungsradius im Bereich von 1,0795 cm (0,425 Zoll) bis 1,3335 cm (0,525 Zoll) aufweist.

7. Zuführungsvorrichtung (305) nach Anspruch 1, wobei eine Spitze-zu-Spitze-Entfernung zwischen zwei oder mehr Haltemerkmalen von gekrümmten Regionen (524) entlang der Länge des Führungsdrahts (515) in einem Bereich von 0,0254 cm (0,0100 Zoll) bis 0,0508 cm (0,0200 Zoll) liegt.

8. Zuführungsvorrichtung (305) nach Anspruch 1, wobei der Führungsdraht (515) wenigstens ein Innenlumen aufweist.

9. Zuführungsvorrichtung (305) nach Anspruch 8, wobei der Führungsdraht (515) wenigstens eine Öffnung entlang einer Länge des Führungsdrahts (515) aufweist, die eine Fluidverbindung zwischen dem Innenlumen des Führungsdrahts (515) und einem den Führungsdraht (515) umgebenden Bereich bereitstellt.

10. Zuführungsvorrichtung (305) nach Anspruch 9, wobei der Bereich um den Führungsdraht (515) herum ein Innenlumen des okularen Implantats (105) und/oder ein Teil des Auges ist.

11. Zuführungsvorrichtung (305) nach Anspruch 10, wobei das Implantat wenigstens ein Loch entlang einer Länge des okularen Implantats (105) aufweist, das Fluidkommunikation zwischen dem Innenlumen des Implantats und einem Bereich um das okulare Implantat (105) herum ermöglicht.

12. Zuführungsvorrichtung (305) nach Anspruch 1, wobei die Zuführungsvorrichtung (305) wenigstens ein Fluidzuführungsmerkmal aufweist, das dem okularen Implantat (105) und/oder dem Auge Fluid von einer Fluidquelle zuführt.

13. Zuführungsvorrichtung (305) nach Anspruch 12, wobei das Fluid von der Fluidquelle eines oder mehrere aus einem Viskoelastikum, einem Arzneimittel und einer Stammzelle beinhaltet.

## Revendications

1. Dispositif de distribution (305) pour distribuer un implant oculaire (105) jusque dans un oeil, le dispositif de distribution comprenant :
une partie formant manche proximale (310) ;
une partie de distribution distale (312) couplée à une extrémité distale de la partie formant manche (310) et configurée pour tenir de manière détachable un implant oculaire (105), l'implant oculaire (105) étant un élément allongé ayant une ou plusieurs lumières internes, la partie de distribution (312) comprenant une gaine (510) positionnée de manière axiale par-dessus un fil de guidage (515), dans lequel le fil de guidage (515) inclut au moins un dispositif de rétention le long d'une longueur du fil de guidage (515) qui aide à retenir l'implant oculaire (105) le long de la longueur du fil de guidage (515), **caractérisé en ce que** l'au moins un dispositif de rétention inclut une configuration sinusoïdale le long d'une longueur du fil de guidage (515), la configuration sinusoïdale comprenant au moins une région courbée (524) qui est configurée pour fournir un ajustement par serrage entre le fil de guidage (515) et une lumière interne de l'implant oculaire (105) ;
un actionneur (420) couplé à un mécanisme qui détache l'implant oculaire (105) de la partie de distribution (312) à l'actionnement de l'actionneur (420), dans lequel le fil de guidage (515) est couplé à une partie du mécanisme qui inclut un ressort (550), dans lequel le mécanisme est configuré pour rétracter le fil de guidage (515) vers la gaine à l'actionnement de l'actionneur (420) ; et
un amortisseur (565) couplé à la partie formant manche (310), l'amortisseur (565) conçu pour amortir la rétraction du fil de guidage (515).

2. Dispositif de distribution (305) selon la revendication 1, dans lequel une extrémité distale du fil de guidage (515) s'étend de manière distale depuis une extrémité distale de la gaine (510) quand le ressort (550) est dans une première formation.

3. Dispositif de distribution (305) selon la revendication 2, dans lequel l'extrémité distale du fil de guidage (515) se rétracte de manière proximale depuis l'extrémité de la gaine (510) quand le ressort (550) est dans une deuxième formation.

4. Dispositif de distribution (305) selon la revendication 3, dans lequel l'actionnement de l'actionneur (420) amène le ressort (550) à effectuer une transition entre la première formation et la deuxième formation.

5. Dispositif de distribution (305) selon la revendication 1, dans lequel une extrémité distale de la gaine (510) soit reçoit soit vient en butée contre une extrémité proximale de l'implant oculaire (105), ou les deux, et empêche l'implant oculaire (105) de glisser dans une direction proximale quand le fil de guidage (515) se rétracte de manière proximale.

6. Dispositif de distribution (305) selon la revendication 1, dans lequel la configuration sinusoïdale inclut un rayon de courbure dans la plage de 1,0795 cm (0,425 pouce) à 1,3335 cm (0,525 pouce).

7. Dispositif de distribution (305) selon la revendication 1, dans lequel une distance de crête à crête entre deux dispositifs de rétention ou régions courbées (524) ou plus, le long de la longueur du fil de guidage (515) est dans une plage de 0,0254 cm (0,0100 pouce) à 0,0508 cm (0,0200 pouce) .

8. Dispositif de distribution (305) selon la revendication 1, dans lequel le fil de guidage (515) inclut au moins une lumière interne.

9. Dispositif de distribution (305) selon la revendication 8, dans lequel le fil de guidage (515) inclut au moins une ouverture le long d'une longueur du fil de guidage (515) qui fournit une communication fluidique entre la lumière interne du fil de guidage (515) et une zone entourant le fil de guidage (515).

10. Dispositif de distribution (305) selon la revendication 9, dans lequel la zone entourant le fil de guidage (515) est soit une lumière interne de l'implant oculaire (105) soit une partie de l'oeil, ou les deux.

11. Dispositif de distribution (305) selon la revendication 10, dans lequel l'implant inclut au moins un trou le long d'une longueur de l'implant oculaire (105) qui fournit une communication fluidique entre la lumière interne de l'implant et une zone entourant l'implant oculaire (105).

12. Dispositif de distribution (305) selon la revendication 1, dans lequel le dispositif de distribution (305) inclut au moins un dispositif de distribution de fluide qui distribue un fluide depuis une source de fluide vers soit l'implant oculaire (105) soit l'oeil ou les deux.

13. Dispositif de distribution (305) selon la revendication 12, dans lequel le fluide depuis la source de fluide inclut un ou plusieurs éléments parmi un produit viscoélastique, un médicament et une cellule-souche.
